(19) Europäisches Patentamt
European Patent Office
Office européen des brevets

(11) Publication number: **0 120 270**
**B1**

(12) # EUROPEAN PATENT SPECIFICATION

(45) Date of publication of patent specification: **27.09.89**

(51) Int. Cl.$^4$: **C 07 D 413/04,** A 61 K 31/42

(21) Application number: **84101614.0**

(22) Date of filing: **17.02.84**

(54) **Furyloxazolylacetic acid derivative, processes for preparing same and pharmaceutical composition.**

(30) Priority: **18.02.83 JP 26814/83**

(43) Date of publication of application:
**03.10.84 Bulletin 84/40**

(45) Publication of the grant of the patent:
**27.09.89 Bulletin 89/39**

(84) Designated Contracting States:
**BE CH DE FR GB IT LI NL**

(56) References cited:
**EP-A-0 087 782**
**GB-A- 818 186**
**GB-A-2 128 184**

(73) Proprietor: **Tanabe Seiyaku Co., Ltd.**
**No. 21 Dosho-machi 3-chome Higashi-ku**
**Osaka-shi Osaka-fu (JP)**

(72) Inventor: **Matsumoto, Kazuo**
**No. 23-27, Yamate-dai 5-chome**
**Ibaraki-shi Osaka-fu (JP)**
Inventor: **Takashima, Kohki**
**No. 1-3-904, Higashi-Kasai 5-chome**
**Edogawa-ku Tokyo-to (JP)**

(74) Representative: **Hansen, Bernd, Dr.rer.nat. et al**
**Hoffmann, Eitle & Partner Patentanwälte**
**Arabellastrasse 4 Postfach 81 04 20**
**D-8000 München 81 (DE)**

**EP 0 120 270 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a furyloxazolylacetic acid derivative, processes for preparing same and pharmaceutical composition.

EP—A—87 782 (published 07.09.1983) discloses furyloxazolylacetic acid derivatives of the formula:

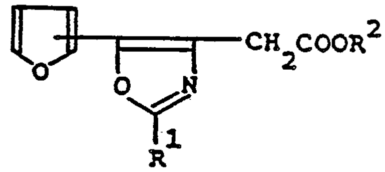

wherein $R^1$ may be a lower alkyl group, and $R^2$ a hydrogen atom or an alkyl group. Such compounds are disclosed as being useful as hypolipidemic agents.

The present invention relates to a furyloxazolylacetic acid derivative of the formula:

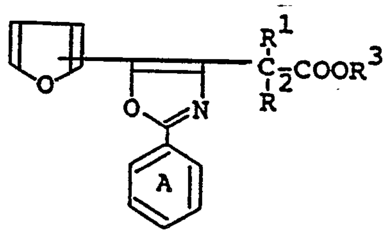

(I)

wherein $R^1$ is $C_1$—$C_4$ alkyl, $R^2$ is hydrogen atom or $C_1$—$C_4$ alkyl, $R^3$ is hydrogen atom or $C_1$—$C_4$ alkyl and ring A is phenyl or halogenophenyl.

The compounds (I) in which $R^3$ is hydrogen can exist in the form of either free acid or a pharmaceutically acceptable salt thereof, and the present invention includes within its scope such pharmaceutically acceptable salt of the compound (I). Besides, the compound (I) in which $R^1$ and $R^2$ are different from each other can exist in the form of two optical isomers (i.e., d- and l-isomers). The present invention also includes within its scope these optical isomers or a mixture thereof.

Hyperlipidemia is known to be one of important causative factors of arteriosclerosis, and various compounds such as dextran sulfate, simfibrate (chemical name: 2-(4-chlorophenoxy)-2-methylpropanoic acid 1,3-propanediyl ester), nicomol (chemical name: nicotinic acid 1,1,3,3-tetraester with 2-hydroxy-1,1,3,3-cyclohexanetetramethanol), clofibrate (chemical name: 2-(4-chlorophenoxy)-2-methylpropanoic acid ethyl ester) and vitamin E nicotinate have been used for the treatment or prophylaxis of said hyperlipidemia.

As a result of investigations, it has now been found that the furyloxazolylacetic acid derivative (I) of the present invention is useful as a hypolipidemic agent. In particular, the furyloxazolylacetic acid derivative (I) shows potent hypolipidemic activity without undesirable side effects such as hepatic dysfunction. Further, the furyloxazolylacetic acid derivative (I) also shows a platelet aggregation-inhibiting activity.

Representative examples of the furyloxazolylacetic acid derivative include those of the formula (I) in which the furyl group is 2-furyl or 3-furyl; $R^1$ is $C_1$—$C_4$ alkyl such as methyl, ethyl, propyl and butyl; each one of $R^2$ and $R^3$ is hydrogen atom or $C_1$—$C_4$ alkyl such as methyl, ethyl, propyl and butyl; and ring A is phenyl or halogenophenyl such as chlorophenyl, fluorophenyl and bromophenyl. Among them, a preferred subgenus is the compound of the formula (I) in which ring A is phenyl, chlorophenyl or fluorophenyl.

Another preferred subgenus is the compound of the formula (I) in which ring A is phenyl, 4-chlorophenyl or 4-fluorophenyl. Further preferred subgenus is the compound of the formula (I) in which $R^1$ is methyl or n-butyl, $R^2$ is hydrogen atom or methyl, $R^3$ is hydrogen atom, ethyl or n-butyl and ring A is phenyl, 4-chlorophenyl or 4-fluorophenyl. Still further preferred subgenus is the compound of the formula (I) in which $R^1$ is methyl, $R^2$ is hydrogen atom or methyl, $R^3$ is hydrogen atom or ethyl, and ring A is 4-chlorophenyl.

According to the present invention, the compound (I) is prepared by

a) alkylating a compound of the formula:

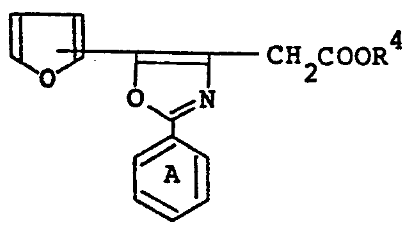

(II)

wherein $R^4$ is $C_1$—$C_4$ alkyl and ring A is the same as defined above, to give a compound of the formula:

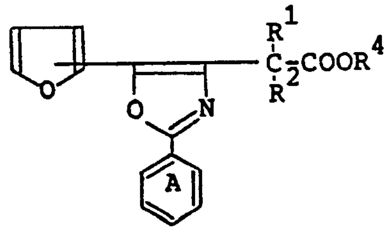

(I-a)

wherein $R^1$, $R^2$, $R^4$ and ring A are the same as defined above, and

b) if required, hydrolyzing the compound (I-a) to give a compound of the formula:

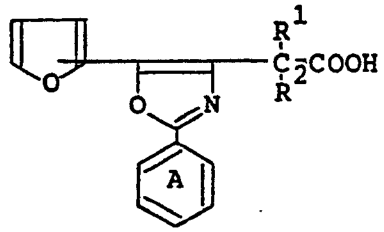

(I-b)

wherein $R^1$, $R^2$ and ring A are the same as defined above.

The alkylation of the compound (II) can be carried out by reacting it with an alkylating agent in a solvent in the pressence of an acid acceptor. Examples of the alkylating agent include $C_1$—$C_4$ alkyl halide such as methyl iodide, ethyl iodide and butyl bromide, and di($C_1$—$C_4$ alkyl) sulfate such as dimethyl sulfate. Examples of the acid acceptor include an alkali metal alkoxide such as sodium ethoxide and potassium t-butoxide; an alkali metal hydride such as sodium hydride; and alkali metal hydroxide such as sodium hydroxide and potassium hydroxide; and an alkyl alkali metal such as butyl lithium. Tetrahydrofuran, dimethylformamide, dimethylsulfoxide, acetonitrile, ethanol, methanol or a mixture thereof are suitable as the solvent. The reaction temperature may vary according to the solvent or the acid acceptor to be used, but in general it is preferred to carry out the reaction at a temperature of −40° to 50°C, especially at a temperature of −40° to 20°C. The compound (I-a) in which $R^1$ is $C_1$—$C_4$ alkyl and $R^2$ is hydrogen atom may be prepared by reacting the compound (II) with an approximately equimolar amount of the alkylating agent. On the other hand, the compound (I-a) in which $R^1$ and $R^2$ are $C_1$—$C_4$ alkyl may be prepared by using about 2 moles, per mole of the compound (II), of the alkylating agent. Alternatively, in preparing the compound (I-a) in which $R^1$ and $R^2$ are $C_1$—$C_4$ alkyl, said alkylation may be carried out by two steps, i.e., reacting the compound (II) with an about equimolar amount of the alkylating agent to give a compound (I-a) in which $R^1$ is $C_1$—$C_4$ alkyl and $R^2$ is hydrogen atom, and then further reacting said compound with the alkylating agent. In the latter case, the compound (I-a) in which $R^1$ and $R^2$ are different each other and are $C_1$—$C_4$ alkyl may be prepared by the use of different alkylating agents in the first and second alkylation steps.

The hydrolysis of the compound (I-a) is accomplished by treating said compound with an acid or an alkali agent in a solvent. The acid includes, for example, mineral acids such as hydrochloric acid or sulfuric acid. On the other hand, the alkali agent includes, for example, alkali metal hydroxides such as sodium hydroxide or potassium hydroxide. Water, alkanol (e.g., methanol, ethanol, propanol), tetrahydrofuran, dioxane and a mixture thereof are suitable as the solvent. It is preferred to carry out the reaction at a temperature of 0° to 100°C, especially at a temperature of 30° to 90°C.

The compound (I) ($R^3$ = H) of the present invention can be used for pharmaceutical use in the form of either free acid or a pharmaceutically acceptable salt thereof.

Pharmaceutically acceptable salts of the compound (I) ($R^3$ = H) include, for example, alkali metal salts such as sodium and potassium salts; alkaline earth metal salts such as calcium and magnesium salts; salts thereof with amino acids such as lysine, ornithine, arginine and histidine salts; and ammonium salt. Such pharmaceutically acceptable salts may be prepared, for example, by neutralizing the free acid with a stoichiometrically equimolar amount of a base such as an alkali metal hydroxide, an alkaline earth metal hydroxide, ammonia or a basic amino acid. The compound (I) and a salt thereof can be administered either orally or parenterally. For oral administration, the compound (I) or a salt thereof may be used in the form of tablets, powder, capsules, granules and the like. Known medicinal excipients such as calcium carbonate, calcium phosphate, corn starch, potato starch, sugar, lactose, talc, magnesium stearate and so forth may be used in making these pharmaceutical preparations. Alternatively, the compound (I) or a salt thereof may be used for oral administration in the form of aqueous or oily suspensions, solutions, syrups or elixirs. On the other hand, injections and suppositories are suitable for parenteral administration of the compound (I) or its salts, and said injections may be made in the form of solutions or suspensions, if required, in conjunction or admixture with distilled water, essential oil (e.g., peanut oil, corn oil) or non-aqueous solvent (e.g., polyethyleneglycol, polypropyleneglycol, lanoline, coconut oil). The daily dose of the compound (I) or a salt thereof may vary depending on the administration route, the age, weight or

conditions of patients, and the severity of diseases to be trated. In general, however, a daily dose of said compound (I) or a salt thereof may be about 0.05 to 100 mg/kg/day, and a preferred dose thereof for oral administration may be 0.1 to 50 mg/kg/day, especially 5 to 20 mg/kg/day.

Concomitantly, the starting compound (II) of the present invention is a novel compound and can be prepared, for example, according to the method shown in the following reaction scheme:

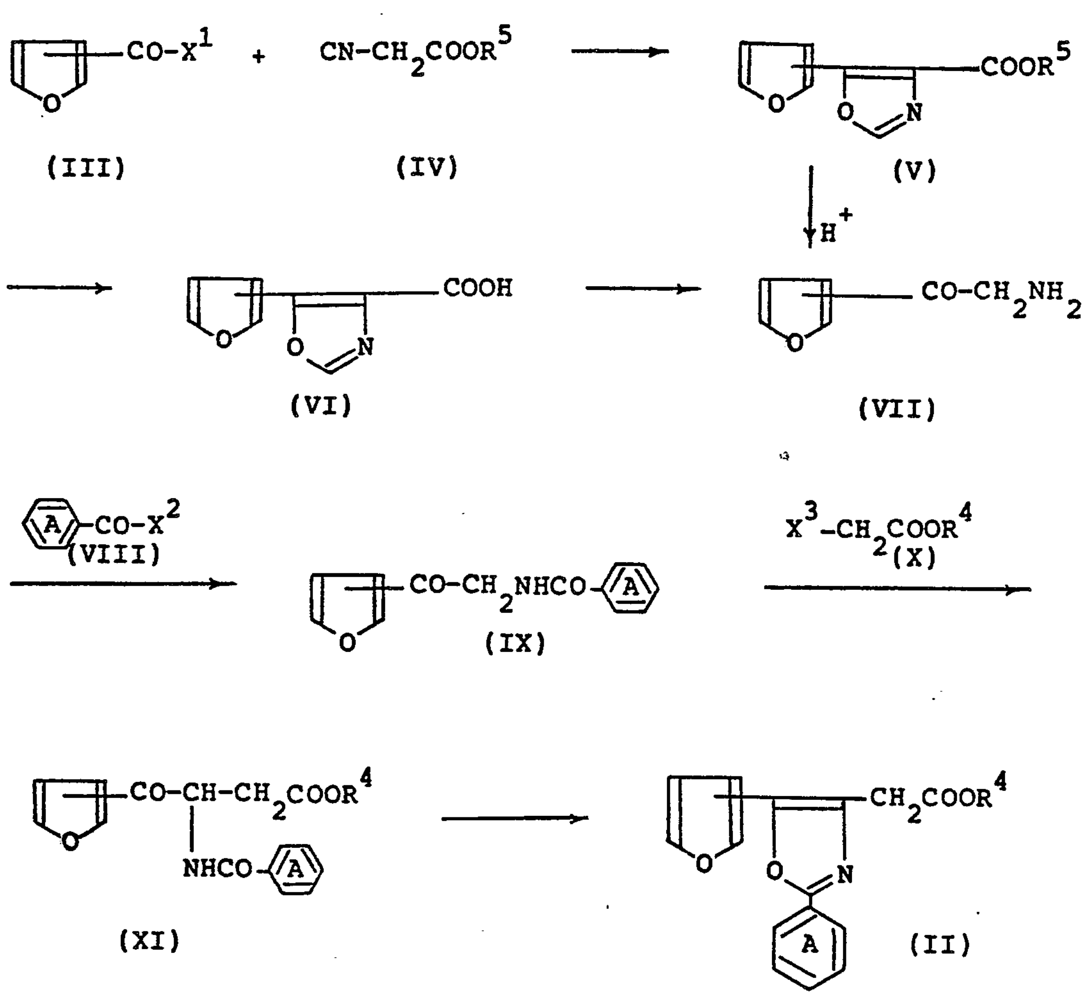


(III) (IV) (V) (VI) (VII) (VIII) (IX) (X) (XI) (II)

wherein $R^5$ is lower alkyl, $X^1$ is halogen atom or a group of the formula:

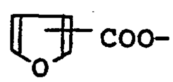
,

$X^2$ and $X^3$ are halogen atom, and $R^4$ and ring A are the same as defined above.

The reaction of the 2- or 3-furancarboxylic acid derivative (III) with the isocyanoacetate compound (IV) is carried out in a solvent (e.g., tetrahydrofuran, dioxane, dimethylformamide) in the presence of a base such as triethylamine, potassium t-butoxide or 1,8-diazabicyclo 5.4.0-undecene-7 at −50° to 50°C. The oxazole derivative (V) thus obtained is saponified with an alkali metal hydroxide (e.g., sodium hydroxide, potassium hydroxide) at 20° to 50°C, and the reaction mixture is acidified to give the oxazolecarboxylic acid derivative (VI) which is then treated with a mineral acid (e.g., 2N hydrochloric acid) at 40° to 80°C to give the aminoketone compound (VII). Alternatively, the aminoketone compound (VII) may also be prepared by hydrolyzing the compound (V) with a mineral acid, for example, by treating the compound (V) with 4N hydrochloric acid at 50° to 80°C. The subsequent reaction of the aminoketone compound (VII) with the acid halide (VIII) is carried out in an aqueous organic solvent (e.g., ethyl acetate, acetone) in the presence of an acid acceptor (e.g., sodium hydroxide, sodium bicarbonate) at −20° to 20°C. The N-acylamine derivative (IX) thus obtained is then reacted with the halogenoacetate compound (X) in a solvent (e.g., tetrahydrofuran, dioxane, dimethylformamide, dimethylsulfoxide, acetonitrile) in the presence of a metallic base (e.g., sodium hydroxide, potassium t-butoxide, n-butyl lithium) at −50° to 30°C to give the 3-acylaminopropionate derivative (XI). The 3-acylaminopropionate derivative (XI) thus obtained is subjected to dehydrative cyclozation in a solvent (e.g., chloroform, methylene chloride, benzene, tetrahydrofuran) in

the presence of a dehydrating agent (e.g., phosphorus oxychloride, phosphorus trichloride, phosphorus pentachloride, thionyl chloride, oxalylchloride, phosphorus pentoxide, sulfuric acid, p-toluene-sulfonic acid, phosgene, acetic anhydride) at −50° to 130°C to give the furyloxazolylacetate derivative (II).

Further, the furyloxazolylacetate derivative (II) may be prepared by the steps of hydrolyzing the compound (II) with an acid or an alkali agent to give the corresponding free acid and then subjecting it to esterification to give the furyloxazolylacetate derivative (II). The hydrolysis or the esterification may be carried out in a conventional manner. For example, the hydrolysis may be carried out by treating the compound (II) with an acid (e.g., hydrochloric acid, sulfuric acid) or an alkali (e.g., sodium hydroxide, potassium hydroxide) in a solvent (e.g., water, methanol, ethanol, tetrahydrofuran, dioxane or a mixture thereof). On the other hand, the esterification may be conducted, for example, by reacting the free acid with an alkanol of the formula: $R^4$—OH (wherein $R^4$ is the same as defined above) in the presence of a halogenating agent (e.g., thionyl chloride, oxalyl chloride, phosphorus tribromide, phosphorus oxychloride, phosphorus trichloride, phosphorus pentachloride).

Practical and presently-preferred embodiments of the present invention are illustratively shown in the following lines.

Experiment 1

(Hypolipidemic activity)

A test compound (50 mg %) was added to a commercial diet, and male SD rats (body weight: 120—140 g, a group of 5 rats) were fed with the diet *ad libitum* for one week. After the experimental period, blood was collected from the tail vein of the rats under ether anesthesia. Then, serum cholesterol and serum triglyceride were measured according to the methods of Zak (Amr. J. Clin., Pathol., Vol. 24, page 1307 (1954)) and Van Handel-Silversmit (J. Lab. & Clin. Med., Vol. 50, page 152 (1957)), respectively. Based on the results obtained above, the decrease (%) in serum cholesterol or triglyceride were calculated by the following formula:

$$\text{Decrease (\%) in serum cholesterol or triglyceride} = \left(1 - \frac{\text{Serum cholesterol or triglyceride (mg/ml) in the medicated group}}{\text{Serum cholesterol or triglyceride (mg/ml) in the control group}}\right) \times 100$$

(Results)

The results are shown in the following Table 1.

Table 1

| Test compound | Decrease (%) in serum choles-terol | Decrease (%) in serum trigly-ceride |
|---|---|---|
| (The compounds of the present invention) | | |
| 2-[2-(4-chlorophenyl)-5-(2-furyl)-4-oxazolyl]-propionic acid | 14 | 34 |
| Ethyl 2-[2-(4-chlorophenyl)-5-(2-furyl)-4-oxazolyl]-propionate | 15 | 22 |
| 2-[2-(4-chlorophenyl)-5-(2-furyl)-4-oxazolyl]-2-methylpropionic acid | 16 | 54 |
| Ethyl 2-[2-(4-chlorophenyl)-5-(3-furyl)-4-oxazolyl]-propionate | 22 | 45 |
| (Positive control) | | |
| Clofibrate | 15 | 16 |

Experiment 2

(Platelet aggregation-inhibiting activity)

Blood was collected from the abdominal aorta of male SD rats (body weight: 250—300 g) which were anesthetized with ether. Nine volumes of said blood were mixed with one volume of an aqueous 3.8% (w/v) trisodium citrate solution, and the mixture was centrifuged at 500 × g for 5 minutes to give platelet-rich plasma (hereinafter referred to as "PRP") as the supernatant solution. The bottom layer was further centrifuged at 1000 × g for 10 minutes to give platelet-poor plasma (hereinafter referred to as "PPP") as the supernatant solution. PRP was diluted with PPP so that the blood platelet count was 8—10 × $10^5$/mm$^3$. Then, a mixture of 200 µl of said diluted PRP and 25 µl of a test compound solution (final concentration: 100 µg/ml) was introduced into a glass cell of SIENCO aggregometer (Sienco Inc., Morrison, Colo. Model DP-247-D). After the mixture was stirred at 1100 rpm at 37°C for 2 minutes, 25 µl of a collagen solution (prepared by Holmsen's method described in Biochim. Biophys. Acta, Vol. 186, page 254 (1969)) was added thereto, and the percentage inhibition of platelet aggregation was calculated in accordance with the following formula from the degree of the platelet aggregation which was estimated by Born's method (Nature, *194*, page 927 (1969)).

$$\text{Percentage inhibition of platelet aggregation} = \left( 1 - \frac{\text{Degree of platelet aggregation which was estimated by adding test compound}}{\text{Degree of platelet aggregation which was estimated without adding test compound}} \right) \times 100$$

Further, on the basis of said percentage inhibition calculated above, the platelet aggregation-inhibiting activity of the test compound was expressed as (−) if the test compound showed less than 10% inhibition of platelet aggregation; or (+) if the test compound showed not less than 10% inhibition of platelet aggregation.

(Results)

The results are shown in the following Table 2.

Table 2

| Test compounds | Platelet aggregation-inhibiting activity |
|---|---|
| (The compounds of the present invention) | |
| Ethyl 2-[2-(4-chlorophenyl)-5-(3-furyl)-4-oxazolyl]-propionate | + |
| Ethyl 2-[2-(4-chlorophenyl)-5-(2-furyl)-4-oxazolyl]-propionate | + |
| (Positive control) | |
| Clofibrate | - |

Example 1

4.8 g of 60% sodium hydride are suspended in 200 ml of dimethylformamide, and a solution of 33.2 g of ethyl 2-[2-(4-chlorophenyl)-5-(2-furyl)-4-oxazolyl]acetate in 200 ml of tetrahydrofuran is added dropwise thereto at −30° to −20°C. Then, 17 g of methyl iodide are added dropwise to the mixture, and the mixture is stirred at −10° to 0°C for 30 minutes. 5 ml of acetic acid are added to the reaction mixture, and the mixture is distilled to remove the solvent. 600 ml of ethyl acetate and 500 ml of water are added to the residue, the mixture is stirred, and then an organic layer is collected therefrom. The organic layer is washed with a

saturated sodium bicarbonate solution and a saturated sodium chloride solution, dried and then condensed. 50 ml of ethanol are added to the residue, and crystalline precipitates are collected by filtration. 30.5 of ethyl 2-[2-(4-chlorophenyl)-5-(2-furyl)-4-oxazolyl]propionate are obtained as colorless needles. Yield: 88%.

M.p. 97—97.5°C.

IR $\nu_{max}^{nujol}$ ($cm^{-1}$): 1740.

NMR ($CDCl_3$): 1.20 (3H, t, J=7.5Hz), 1.63 (3H, d, J=7.5Hz), 3.8—4.4 (3H, m), 6.43 (1H, dd, J=3.5 and 1.5Hz), 6.65 (1H, d, J=3.5Hz), 7.32 and 7.92 (2H, each $A_2'$ $B_2'$, J=8Hz), 7.45 (1H, dd, J=1.5Hz).

Mass (m/e): 345 ($M^+$).

Example 2

5.0 g of n-butyl 2-[2-(4-chlorophenyl)-5-(2-furyl)-4-oxazolyl]acetate, 30 ml of tetrahydrofuran, 0.8 g of sodium hydride, 50 ml of dimethylformamide and 2.5 g of methyl iodide are treated in the same manner as described in Example 1. 5.2 g of n-butyl 2-[2-(4-chlorophenyl)-5-(2-furyl)-4-oxazolyl]propionate are thereby obtained as needles. Yield: 93%.

M.p. 48—50°C.

IR $\nu_{max}^{nujol}$ ($cm^{-1}$): 1735.

Mass (m/e): 373 ($M^+$, 100).

Example 3

1.65 g of ethyl 2-[2-(4-chlorophenyl)-5-(3-furyl)-4-oxazolyl]acetate, 10 ml of tetrahydrofuran, 0.25 g of sodium hydride, 30 ml of dimethylformamide and 0.84 g of methyl iodide are treated in the same manner as described in Example 1. 1.31 g of ethyl 2-[2-(4-chlorophenyl)-5-(3-furyl)-4-oxazolyl]propionate are thereby obtained. Yield: 73%.

M.p. 108—109°C.

IR $\nu_{max}^{nujol}$ ($cm^{-1}$): 1735.

Mass (m/e): 345 ($M^+$, 90).

Example 4

1.57 g of ethyl 2-[2-(4-fluorophenyl)-5-(2-furyl)-4-oxazolyl]acetate and 0.78 g of methyl iodide are dissolved in 3 ml of tetrahydrofuran, and the solution is added dropwise to 10 ml of an ethanol solution containing 0.41 g of sodium ethoxide. Said dropwise addition is carried out at 5°—10°C. Then, the mixture is treated in the same manner as described in Example 1. 0.74 g of ethyl 2-[2-(4-fluorophenyl)-5-(2-furyl)-4-oxazolyl]propionate are therbey obtained as needles. Yield: 45%.

M.p. 95—96°C.

IR $\nu_{max}^{nujol}$ ($cm^{-1}$): 1735.

Mass (m/e): 329 ($M^+$, 100).

Example 5

4.97 g of ethyl 2-[2-(4-chlorophenyl)-5-(2-furyl)-4-oxazolyl]acetate are dissolved in a mixture of 50 ml of dimethylformamide and 30 ml of tetrahydrofuran. 2.16 g of 50% sodium hydride are added to the mixture at −15°C. The mixture is stirred for 5 minutes, and then 1.86 ml of methyl iodide are added thereto. After the temperature of the mixture is raised gradually to room temperature, the mixture is stirred at the same temperature for 5 hours. 1 ml of acetic acid is added to the mixture, and the mixture is distilled to remove tetrahydrofuran. 300 ml of ethyl acetate and 200 ml of water are added to the residue. The mixture is shaken, and the organic layer is collected. The organic layer is washed with a saturated sodium bicarbonate solution and a saturated sodium chloride solution, dried and then condensed. 20 ml of ethanol are added to the residue, and the crystalline precipitates are collected by filtration. 4.50 g of ethyl 2-[2-(4-chlorophenyl)-5-(2-furyl)-4-oxazolyl]-2-methylpropionate are obtained as colorless needles. Yield: 84%.

M.p. 89—90°C.

IR $\nu_{max}^{nujol}$ ($cm^{-1}$): 1730.

NMR ($CDCl_3$): 1.07 (3H, t, J=7Hz), 1.68 (6H, s), 4.02 (2H, q, J=7Hz), 6.44 (1H, dd, J=2 and 4Hz), 6.59 (1H, d, J=4Hz), 7.34 and 7.93 (2H, each, $A_2'B_2'$, J=9Hz), 7.44 (1H, d, J=2Hz).

Mass (m/e): 359 ($M^+$, 90).

Example 6

3.32 g of ethyl 2-[2-(4-chlorophenyl)-5-(3-furyl)-4-oxazolyl]acetate, 50 ml of dimethylformamide, 20 ml of tetrahydrofuran, 0.50 g of 50% sodium hydride and 1.70 g of methyl iodide are treated in the same manner as described in Example 5. 2.7 g of ethyl 2-[2-(4-chlorophenyl)-5-(3-furyl)-4-oxazolyl]-2-methylpropionate are thereby obtained as needles. Yield: 75%.

M.p. 71—72°C.

IR $\nu_{max}^{nujol}$ ($cm^{-1}$): 1720.

Mass (m/e): 359 ($M^+$, 70).

Example 7

1.70 g of ethyl 2-[2-(4-chlorophenyl)-5-(2-furyl)-4-oxazolyl]propionate are dissolved in a mixture of 10 ml of water and 50 ml of methanol, and 1.12 g of potassium hydroxide are added thereto. The mixture is stirred at 60°C for one hour. The reaction mixture is condensed to remove methanol, and 30 ml of water are added to the residue. The aqueous solution is washed with ether, and the solution is acidified to pH 3 with 10% hydrochloric acid. The aqueous solution is extracted with ethyl acetate, and the extract is dried and then condensed. Diisopropyl ether is added to the residue, and the crystalline precipitates are collected by filtration. 1.0 g of 2-[2-(4-chlorophenyl)-5-(2-furyl)-4-oxazolyl]propionic acid is obtained. Yield: 64%.

M.p. 186—187°C.

IR $\nu_{max}^{nujol}$ ($cm^{-1}$): 1710.

NMR ($CDCl_3$-DMSO-$d_6$): 1.6 (3H, d, J=7.5Hz), 4.12 (1H, q, J=7.5Hz), 6.47 (1H, m), 7.1—8.1 (6H, m).

Mass (m/e): 317 ($M^+$, 20).

Example 8

A mixture of 2.0 g of ethyl 2-[2-(4-chlorophenyl)-5-(2-furyl)-4-oxazolyl]-2-methylpropionate, 0.47 g of potassium hydroxide, 30 ml of methanol and 10 ml of water is refluxed for 5 hours on a water bath. Then, the reaction mixture is treated in the same manner as described in Example 7. 1.58 g of 2-[2-(4-chlorophenyl)-5-(2-furyl)-4-oxazolyl]-2-methylpropionic acid are thereby obtained as needles. Yield: 86%.

M.p. 186—189°C.

IR $\nu_{max}^{nujol}$ ($cm^{-1}$): 1720.

NMR (DMSO-$d_6$): 1.61 (6H, s), 6.6—7.0 (2H, m), 7.52 and 7.98 (2H each $A_2'B_2'$, J=8Hz), 7.83 (1H, broad s).

Mass (m/e): 331 ($M^+$, 60).

Example 9

0.14 g of 60% sodium hydride is suspended in 20 ml of tetrahydrofuran, and a mixture of 3.3 g of ethyl 2-[2-(4-chlorophenyl)-5-(2-furyl)-4-oxazolyl]acetate, 1.6 g of n-butyl bromide and 10 ml of dimethylformamide is added dropwise thereto at −40° to −20°C. After the temperature of the mixture is raised gradually to room temperature, the mixture is stirred at the same temperature for 2 hours. Then, the reaction mixture is treated in the same manner as described in Example 1. 2.28 g of ethyl 2-[2-(4-chlorophenyl)-5-(2-furyl)-4-oxazolyl]hexanoate are thereby obtained.

M.p. 76—78°C.

IR $\nu_{max}^{nujol}$ ($cm^{-1}$): 1730.

Mass (m/e): 387 ($M^+$, 100).

Example 10

0.096 g of 50% sodium hydride is suspended in 10 ml of dimethylformamide, and a mixture of 0.65 g of n-butyl 2-[2-phenyl-5-(2-furyl)-4-oxazolyl]acetate, 0.31 g of methyl iodide and 10 ml of tetrahydrofuran is added dropwise thereto at −30° to −10°C. After the temperature of the mixture is raised gradually to room temperature, the mixture is stirred at the same temperature for 30 minutes. Then, the reaction mixture is treated in the same manner as described in Example 1. 0.50 g of n-butyl 2-[2-phenyl-5-(2-furyl)-4-oxazolyl]-propionate are thereby obtained. Yield: 74%.

M.p. 43—44°C.

IR $\nu_{max}^{nujol}$ ($cm^{-1}$): 1735.

Mass (m/e): 339 ($M^+$, 60).

(Preparation of the starting compounds)

Preparation 1

(1) To a mixture of 10 g of (2-furylcarbonyl)methylamine hydrochloride, 12.5 g of sodium bicarbonate, 200 ml of ethyl acetate and 100 ml of water are dropwise added 12.0 g of 4-chlorobenzoyl chloride at a temperature below 10°C under stirring. The mixture is stirred at room temperature for 3 hours. Then, the ethyl acetate layer is collected, washed with water and dried. Said ethyl acetate layer is evaporated under reduced pressure to remove solvent. 16.0 g of N-(4-chlorobenzoyl)-(2-furylcarbonyl)methylamine are thereby obtained. Yield: 98.2%.

M.p. 138°—139°C.

IR $\nu_{max}^{nujol}$ ($cm^{-1}$): 3400, 1680, 1660, 1595.

(2) To a solution of 40 g of N-(4-chlorobenzoyl)-(2-furylcarbonyl)methylamine in 160 ml of dimethylformamide are added 7.2 g of 61% sodium hydride at −50° to −40°C under stirring. The mixture is further stirred at the same temperature for 5 minutes. 27.9 g of ethyl bromoacetate are added to the mixture at −50° to −40°C, and said mixture is stirred at the same temperature for one hour and further stirred at about 0°C for one hour. After the reaction is completed, the mixture is neutalized with acetic acid. Then, water is added to the mixture and said mixture is extracted with ethyl acetate. The extract is washed with an aqueous sodium bicarbonate solution and water, successively.

Said extract is dried and then evaporated under reduced pressure to remove solvent. The residue is

crystallized with a mixture of isopropyl ether and n-hexane, whereby 37.0 g of ethyl 3-(4-chlorobenzoylamino)-3-(2-furylcarbonyl)propionate are obtained. Yield: 70%.

M.p. 100°—102°C.

IR $\nu_{max}^{nujol}$ ($cm^{-1}$): 3300, 3100, 1738, 1680, 1630, 1595.

(3) 37 g of ethyl 3-(4-chlorobenzoylamino)-3-(2-furylcarbonyl)propionate are dissolved in 150 ml of chloroform. 64.9 g of phosphorus oxychloride are added dropwise to the solution at room temperature, and the mixture is refluxed at 60° to 70°C for 8 hours under stirring. After the reaction is completed, the mixture is poured into ice-water. The aqueous mixture is neutralized with sodium bicarbonate and then extracted with ethyl acetate. The extract is washed with water, dried, treated with activated charcoal and then evaporated under reduced pressure to remove solvent. The residue is crystallized with diisopropyl ether, and then recrystallized from ethanol. 30 g of ethyl 2-[2-(4-chlorophenyl)-5-(2-furyl)-4-oxazolyl]acetate are thereby obtained. Yield: 85.5%.

M.p. 105°—106°C.

IR $\nu_{max}^{nujol}$ ($cm^{-1}$): 1723, 1600.

Preparation 2

(1) 6.0 g of potassium tert-butoxide are dissolved in 60 ml of tetrahydrofuran, and 4.4 g of methyl α-isocyanoacetate are added dropwise thereto at −50°C under stirring. The mixture is stirred at the same temperature for one hour. Then, 5.8 g of 3-furylcarbonyl chloride are added dropwise to the mixture at −50°C under stirring, and said mixture is further stirred for 2 hours. After the reaction is completed, the mixture is adjusted to a pH of 3 to 4 with acetic acid and then evaporated under reduced pressure to remove solvent. The residue is extracted with ethyl acetate, and the extract is washed with water and an aqueous sodium bicarbonate solution, successively. Said extract is dried and evaporated under reduced pressure to remove solvent. The residue is washed with isopropyl ether, whereby 6.5 g of methyl 5-(3-furyl)oxazole-4-carboxylate are obtained. Yield: 75.4%.

M.p. 87°—88°C.

IR $\nu_{max}^{nujol}$ ($cm^{-1}$): 3120, 1695.

(2) 3 g of methyl 5-(3-furyl)oxazole-4-carboxylate and 2.6 g of 85% potassium hydroxide are dissolved in 100 ml of methanol, and the solution is stirred at room temperature overnight. Then, the reaction mixture is evaporated under reduced pressure to remove methanol, and the residue is acidified with dil. hydrochloric acid. The precipitated crystals are collected by filtration, washed with cold water and then dried. 2.6 g of 5-(3-furyl)oxazole-4-carboxylic acid are thereby obtained. Yield: 93.7%.

M.p. 189°—191°C.

IR $\nu_{max}^{nujol}$ ($cm^{-1}$): 3110, 1700, 1600.

(3) A mixture of 3.0 g of 5-(3-furyl)oxazole-4-carboxylic acid and 50 ml of 3N hydrochloric acid is stirred at 40° to 50°C for 6.5 hours. Then, the mixture is condensed under reduced pressure, and the residue is crystallized with acetone. The precipitated crystals are collected by filtration, whereby 2.2 g of (3-furylcarbonyl)methylamine hydrochloride are obtained. Yield: 80%.

M.p. 196°—198°C.

IR $\nu_{max}^{nujol}$ ($cm^{-1}$): 3110, 1680, 1560.

(4) 5 g of (3-furylcarbonyl)methylamine hydrochloride, 6.0 g of sodium bicarbonate and 6.0 g of 4-chlorobenzoyl chloride are treated in the same manner as described in Preparation 1-(1). 8.2 g of N-(4-chlorobenzoyl)-(3-furylcarbonyl)methylamine are thereby obtained. Yield: 100%.

M.p. 152°—154°C.

IR $\nu_{max}^{nujol}$ ($cm^{-1}$): 3350, 3110, 1675, 1635, 1595.

(5) 8.3 g of N-(4-chlorobenzoyl)-(3-furylcarbonyl)methylamine, 1.5 g of 61% sodium hydride and 5.8 g of ethyl bromoacetate are treated in the same manner as described in Preparation 1-(2). 8.5 g of ethyl 3-(4-chlorobenzoylamino)-3-(3-furylcarbonyl)propionate are thereby obtained. Yield: 77.3%.

M.p. 93°—95°C (decomp.).

IR $\nu_{max}^{nujol}$ ($cm^{-1}$): 3340, 3100, 1705, 1670, 1640.

(6) 7.0 g of ethyl 3-(4-chlorobenzoylamino)-3-(3-furylcarbonyl)propionate, 35 ml of chloroform and 12.3 g of phosphorus oxychloride are treated in the same manner as described in Preparation 1-(3). 5.7 g of ethyl 2-[2-(4-chlorophenyl)-5-(3-furyl)-4-oxazolyl]acetate are thereby obtained. Yield: 85.5%.

M.p. 124°—125°C.

IR $\nu_{max}^{nujol}$ ($cm^{-1}$): 3130, 1725.

Preparation 3

(1) A solution of 0.4 g of potassium hydroxide in 30 ml of methanol is added to 1.0 g of ethyl 2-[2-(4-chlorophenyl)-5-(2-furyl)-4-oxazolyl]acetate at 5° to 10°C. The mixture is stirred at room temperature for 10

hours. Then, the precipitates are collected by filtration, washed with ether and then dried. 0.85 g of potassium 2-[2-(4-chlorophenyl)-5-(2-furyl)-4-oxazolyl]acetate is obtained. Yield: 80.2%.

M.p. >250°C.

IR $\nu_{max}^{nujol}$ ($cm^{-1}$): 1610.

(2) 0.85 g of potassium 2-[2-(4-chlorophenyl)-5-(2-furyl)-4-oxazoyl]acetate is dissolved in 20 ml of water, and the solution is acidified with hydrochloric acid. The precipitates are collected by filtration and then recrystallized from ethanol. 0.69 g of 2-[2-(4-chlorophenyl)-5-(2-furyl)-4-oxazolyl]acetic acid is thereby obtained. Yield: 90.9%.

M.p. 191°—194°C (decomp.).

IR $\nu_{max}^{nujol}$ ($cm^{-1}$): 1720.

(3) To a soloution of 2.0 g of 2-[2-(4-chlorophenyl)-5-(2-furyl)-4-oxazolyl]acetic acid in 20 ml of chloroform are added 2.0 g of thionyl chloride at room temperature. The mixture is refluxed for 3 hours under stirring. After cooling, 0.6 g of n-butanol is added to the mixture, and said mixture is stirred at room temperature for 6 hours. After the reaction is completed, the mixture is evaporated under reduced pressure to remove solvent. The residue is extracted with ethyl acetate, and the extract is washed with an aqueous sodium bicarbonate solution and water, successively. Then the extract is dried and then evaporated under reduced pressure to remove solvent. The residue is recrystallized from ethanol, whereby 1.5 g of n-butyl 2-[2-(4-chlorophenyl)-5-(2-furyl)-4-oxazolyl]acetate are obtained. Yield: 62.3%.

M.p. 96°—97°C.

IR $\nu_{max}^{nujol}$ ($cm^{-1}$): 3050, 1730.

Preparation 4

(1) 23.4 g of (2-furylcarbonyl)methylamine hydrochloride, 29.3 g of sodium bicarbonate and 23.0 g of 4-fluorobenzoyl chloride are treated in the same manner as described in Preparation 1-(1). 24.0 g of N-(4-fluorobenzoyl)-(2-furylcarbonyl)methylamine are thereby obtained. Yield: 66.8%.

M.p. 153°—155°C.

IR $\nu_{max}^{nujol}$ ($cm^{-1}$): 3410, 3100, 1680, 1655.

(2) 24.0 g of N-(4-fluorobenzoyl)-(2-furylcarbonyl)methylamine, 4.7 g of 61% sodium hydride and 18.4 g of ethyl bromoacetate are treated in the same manner as described in Preparation 1-(2). 21.0 g of ethyl 3-(4-fluorobenzoylamino)-3-(2-furylcarbonyl)propionate are thereby obtained. Yield: 64.8%.

M.p. 95.5°—97°C.

IR $\nu_{max}^{nujol}$ ($cm^{-1}$): 3300, 1725, 1690, 1640, 1600.

(3) 6.67 g of ethyl 3-(4-fluorobenzoylamino)-3-(2-furylcarbonyl)propionate, 35 ml of chloroform and 12.3 g of phosphorus oxychloride are treated in the same manner as described in Preparation 1-3. 4.98 g of ethyl 2-[2-(4-fluorophenyl)-5-(2-furyl)-4-oxazolyl]acetate are thereby obtained. Yield: 78.9%.

M.p. 110°—111°C.

IR $\nu_{max}^{nujol}$ ($cm^{-1}$): 3110, 1725.

Preparation 5

(1) 3.4 g of (2-furylcarbonyl)methylamine hydrochloride, 4.4 g of sodium bicarbonate and 3.3 g of benzoyl chloride are treated in the same manner as described in Preparation 1-(1). 4.0 g of N-benzoyl-(2-furylcarbonyl)methylamine are thereby obtained. Yield: 83.3%.

M.p. 132°—133°C.

IR $\nu_{max}^{nujol}$ ($cm^{-1}$): 3400, 3100, 1675, 1600.

(2) 4.0 g of N-benzoyl-(2-furylcarbonyl)methylamine, 0.8 g of 61% sodium hydride and 3.2 g of ethyl bromoacetate are treated in the same manner as described in Preparation 1-(2). 4.0 g of ethyl 3-benzoylamino-3-(2-furylcarbonyl)propionate are thereby obtained. Yield: 72.7%.

M.p. 75°—76°C.

IR $\nu_{max}^{nujol}$ ($cm^{-1}$): 3300, 1715, 1680, 1640.

(3) 6.31 g of ethyl 3-benzoylamino-3-(2-furylcarbonyl)propionate, 35 ml of chloroform and 12.3 g of phosphorus oxychloride are treated in the same manner as described in Preparation 1-(3). 4.47 g of ethyl 2-[2-phenyl-5-(2-furyl)-4-oxazolyl]acetate are thereby obtained. Yield: 75%.

M.p. 79°—80°C.

IR $\nu_{max}^{nujol}$ ($cm^{-1}$): 3130, 1735.

Preparation 6

(1) 3.5 g of ethyl 2-[2-phenyl-5-(2-furyl)-4-oxazolyl]acetate are dissolved in a mixture of 50 ml of methanol and 10 ml of water, and 1.2 g of sodium hydroxide are added thereto. The mixture is stirred at

room temperature for 10 hours. After the reaction is completed, the mixture is condensed under reduced pressure to removal methanol. Water is added to the residue, and the aqueous mixture is adjusted to ph 2 with conc. hydrochloric acid, and then extracted with ethyl acetate. The extract is washed with water, dried and then evaporated under reduced pressure to remove solvent. The residue is recrystallized from ethanol, whereby 2.5 g of 2-[2-phenyl-5-(2-furyl)-4-oxazolyl]acetic acid are obtained. Yield 78.1%.

M.p. 175°—177°C.

IR $\nu_{max}^{nujol}$ (cm$^{-1}$): 3100, 1690.

(2) 5.86 g of 2-[2-phenyl-5-(2-furyl)-4-oxazolyl]acetic acid, 50 ml of chloroform, 4.8 g of thionyl chloride and 2.5 g of n-butanol are treated in the same manner as described in Preparation 3-(3). 4.22 g of n-butyl 2-[2-phenyl-5-(2-furyl)-4-oxazolyl]acetate are thereby obtained. Yield: 64.9%.

M.p. 53—54°C.

IR $\nu_{max}^{nujol}$ (cm$^{-1}$): 1720.

Mass (m/e): 325 (M$^+$, 75).

## Claims

1. A compound of the formula:

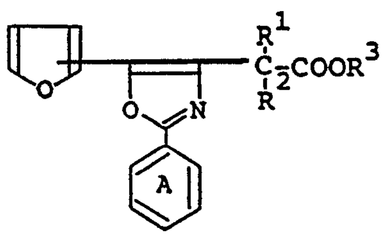

wherein $R^1$ is an alkyl group having 1 to 4 carbon atoms, $R^2$ is hydrogen atom or an alkyl group having 1 to 4 carbon atoms, $R^3$ is hydrogen atom or an alkyl group having 1 to 4 carbon atoms and ring A is phenyl or halogenophenyl.

2. A compound according to claim 1, in which $R^3$ is a hydrogen atom or a pharmaceutically acceptable salt thereof.

3. A compound according to claim 1 or claim 2, in which ring A is phenyl, chlorophenyl or fluorophenyl.

4. A compound according to any preceding claim, in which ring A is phenyl, 4-chlorophenyl or 4-fluorophenyl.

5. A compound according to any preceding claim, in which $R^1$ is methyl or n-butyl, $R^2$ is a hydrogen atom or methyl and $R^3$ is hydrogen atom, ethyl or n-butyl.

6. A compound according to claim 5, in which $R^1$ is methyl, $R^3$ is a hydrogen atom or ethyl and ring A is 4-chlorophenyl.

7. The compound of claim 6 which is ethyl 2-[2-(4-chlorophenyl)-5-(3-furyl)-4-oxazolyl]propionate.

8. The compound of claim 6 which is 2-[2-(4-chlorophenyl)-5-(2-furyl)-4-oxazolyl]-2-methyl-propionic acid or a pharmaceutically acceptable salt thereof.

9. The compound of claim 6 which is ethyl 2-[2-(4-chlorophenyl)-5-(2-furyl)-4-oxazolyl]propionate.

10. The compound of claim 6 which is 2-[2-(4-chlorophenyl)-5-(2-furyl)-4-oxazolyl]propionic acid or a pharmaceutically acceptable salt thereof.

11. A process for preparing a compound of the formula

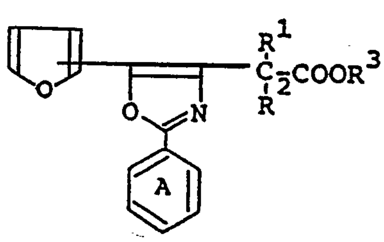

(I)

wherein $R^1$, $R^2$, $R^3$ and ring A are as defined in claim 1, which comprises

a) alkylating a compound of the formula

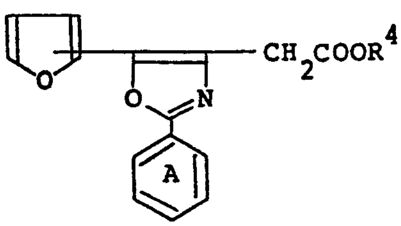

(II)

wherein $R^4$ is an alkyl group having 1 to 4 carbon atoms and ring A is the same as defined above, to give a compound of the formula

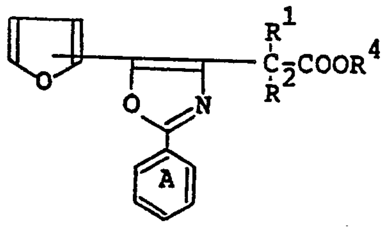

(I-a)

wherein $R^1$, $R^2$, $R^4$ and ring A are the same as defined above, and

b) if required, hydrolyzing the compound (I-a) to give a compound of the formula

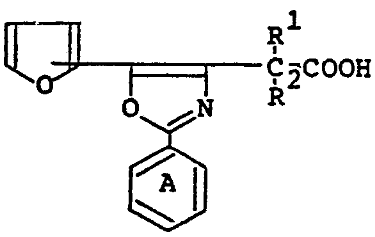

(I-b)

wherein $R^1$, $R^2$ and ring A are as defined above.

12. A process according to claim 11, wherein the compound of the formula (I-b) is further converted into a pharmaceutically acceptable salt thereof.

13. A pharmaceutical composition which comprises a therapeutically effective amount of a compound according to any of claims 1 to 10 and a pharmaceutically acceptable carrier therefor.

14. A pharmaceutical composition according to claim 13 wherein $R^3$ is a hydrogen atom.

**Patentansprüche**

1. Verbindung der Formel

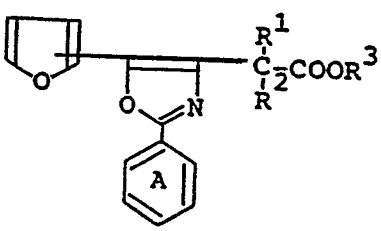

wobei $R^1$ eine Alkylgruppe mit 1 bis 4 Kohlenstoffatomen, $R^2$ ein Wasserstoffatom oder eine Alkylgruppe mit 1 bis 4 Kohlenstoffatomen, $R^3$ ein Wasserstoffatom oder eine Alkylgruppe mit 1 bis 4 Kohlenstoffatomen und der Ring A Phenyl oder Halogenphenyl ist.

2. Verbindung nach Anspruch 1, in der $R^3$ ein Wasserstoffatom oder ein pharmazeutisch verträgliches Salz davon ist.

3. Verbindung nach Anspruch 1 oder 2, in der der Ring A Phenyl, Chlorphenyl oder Fluorphenyl ist.

4. Verbindung nach einem der vorhergehenden Ansprüche, in der der Ring A Phenyl, 4-Chlorphenyl oder 4-Fluorphenyl ist.

5. Verbindung nach einem der vorhergehenden Ansprüche, in der $R^1$ Methyl oder n-Butyl, $R^2$ ein Wasserstoffatom oder Methyl, und $R^3$ ein Wasserstoffatom, Ethyl oder n-Butyl ist.

6. Verbindung nach Anspruch 5, in der $R^1$ Methyl, $R^3$ ein Wasserstoffatom oder Ethyl und der Ring A 4-Chlorphenyl ist.

7. Verbindung nach Anspruch 6, die Ethyl-2-[2-(4-chlorphenyl)-5-(3-furyl)-4-oxazolyl]-propionat ist.

8. Verbindung nach Anspruch 6, die 2-[2-(4-Chlorphenyl)-5-(2-furyl-4-oxazolyl]-2-methyl-propionsäure oder ein pharmazeutisch verträgliches Salz davon ist.

9. Verbindung nach Anspruch 6, die Ethyl-2-[2-(4-chlorphenyl)-5-(2-furyl)-4-oxazolyl]-propionat ist.

10. Verbindung nach Anspruch 6, die 2-[2-(4-Chlorphenyl)-5-(2-furyl)-4-oxazolyl]-propionsäure oder ein pharmazeutisch verträgliches Salz davon ist.

11. Verfahren zur Herstellung einer Verbindung der Formel

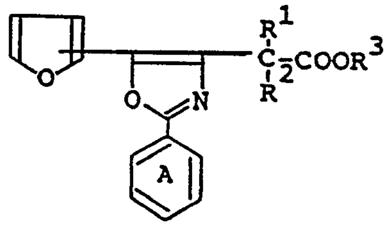

(I)

wobei $R^1$, $R^2$, $R^3$ und der Ring A wie in Anspruch 1 definiert sind, das umfasst:

(a) Alkylieren einer Verbindung der Formel

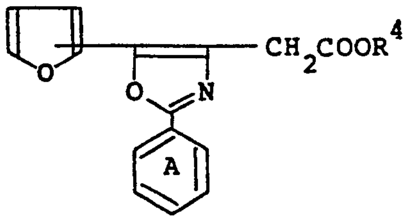

(II)

wobei $R^4$ eine Alkylgruppe mit 1 bis 4 Kohlenstoffatomen ist, und der Ring A wie oben definiert ist, unter Erhalt einer Verbindung der Formel

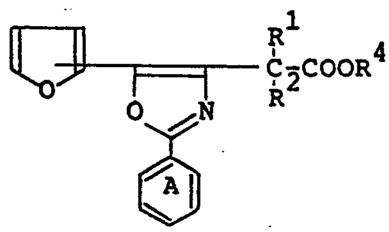

(Ia)

wobei $R^1$, $R^2$, $R^4$ und der Ring A wie oben definiert sind, und

(b) falls notwendig, Hydrolysieren der Verbindung (Ia) unter Erhalt einer Verbindung der Formel

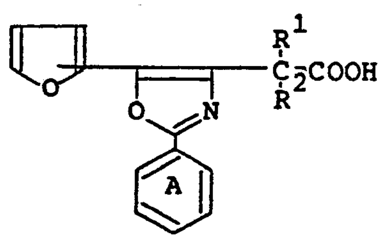

(Ib)

wobei $R^1$, $R^2$ und der Ring A wie oben definiert sind.

12. Verfahren nach Anspruch 11, wobei die Verbindung der Formel (Ib) des weiteren in ein pharmazeutisch verträgliches Salz davon umgewandelt wird.

13. Pharmazeutische Zusammensetzung, die eine therapeutisch wirksame Menge einer Verbindung gemäss einem der Ansprüche 1 bis 10 und einen dafür pharmazeutisch verträglichen Träger umfasst.

14. Pharmazeutische Zusammensetzung nach Anspruch 13, wobei $R^3$ ein Wasserstoffatom ist.

**Revendications**

1. Un composé de formule:

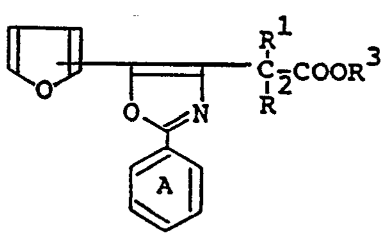

dans laquelle $R^1$ est un groupement alkyle comportant 1 à 4 atomes de carbone, $R^2$ est un atome

d'hydrogène ou un groupement alkyle comportant 1 à 4 atomes de carbone, $R^3$ est un atome d'hydrogène ou un groupement alkyle comportant 1 à 4 atomes de carbone et le cycle A est phényle ou halogénophényle.

2. Un composé selon la revendication 1, dans lequel $R^3$ est un atome d'hydrogène ou un de ses sels pharmaceutiquement acceptable.

3. Un composé selon la revendication 1 ou 2, dans lequel le cycle A est phényle, chlorophényle ou fluorophényle.

4. Un composé selon l'une quelconque des revendications précédentes, dans lequel le cycle A est phényle, 4-chlorophényle ou 4-fluorophényle.

5. Un composé selon l'une quelconque des revendications précédentes, dans lequel $R^1$ est un groupement méthyle ou n-butyle, $R^2$ est un atome d'hydrogène ou un groupement méthyle et $R^3$ est un atome d'hydrogène, un groupement éthyle ou n-butyle.

6. Un composé selon la revendication 5, dans lequel $R^1$ est un groupement méthyle, $R^3$ est un atome d'hydrogène ou un groupement éthyle et le cycle A est un 4-chlorophényle.

7. Le composé de la revendication 6, qui est le 2-[2-(4-chlorophényl)-5-(3-furyl)-4-oxazolyl]-propionate d'éthyle.

8. Le composé de la revendication 6, qui est l'acide 2-[2-(4-chlorophényl)-5-(2-furyl)-4-oxazolyl]-2-méthylpropionique ou un de ses sels pharmaceutiquement acceptable.

9. Le composé de la revendication 6, qui est le 2-[2-(4-chlorophényl)-5-(2-furyl)-4-oxazolyl]-propionate d'éthyle.

10. Le composé de la revendication 6, qui est l'acide 2-[2-(4-chlorophényl)-5-(2-furyl)-4-oxazolyl]-propionique ou un de ses sels pharmaceutiquement acceptable.

11. Un procédé pour préparer un composé de formule:

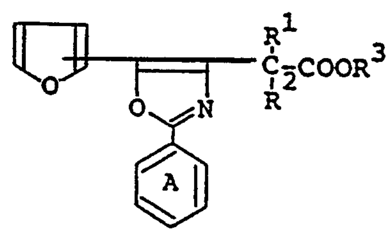

(I)

dans laquelle $R^1$, $R^2$, $R^3$ et le cycle A sont tels que définis dans la revendication 1, qui comprend

a) l'alkylation d'un composé de formule:

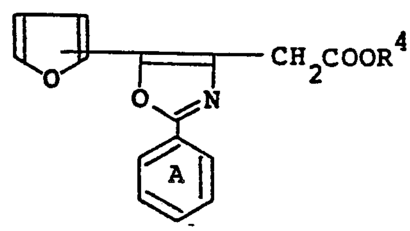

(II)

dans laquelle $R^4$ est un groupement alkyle comportant 1 à 4 atomes de carbone et le cycle A est tel que défini ci-dessus, pour donner un composé de formule:

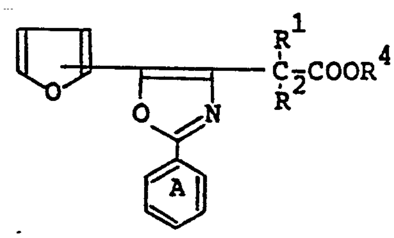

(Ia)

dans laquelle $R^1$, $R^2$, $R^4$ et le cycle A sont tels que définis ci-dessus, et

b) si nécessaire, l'hydrolyse du composé (I-a) pour donner un composé de formule:

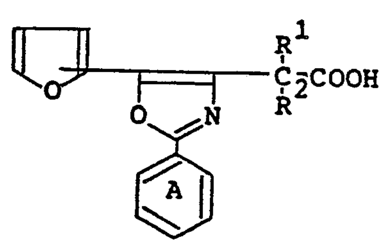

(Ib)

dans laquelle $R^1$, $R^2$ et le cycle A sont tels que définis ci-dessus.

12. Un procédé selon la revendication 11, dans lequel on convertit ultérieurement le composé de formule (I-b) en un de ses sels pharmaceutiquement acceptable.

13. Une composition pharmaceutiquement qui comprend une quantité thérapeutiquement efficace d'un composé selon l'une quelconque des revendications 1 à 10 et un véhicule pharmaceutiquement acceptable pour ce composé.

14. Une composition pharmaceutiquement selon la revendication 13, dans laquelle $R^3$ est un atome d'hydrogène.